# EUROPEAN PATENT APPLICATION

(11) **EP 4 104 842 A1**
(43) Date of publication of application: **21.12.2022**
(21) Application number: 21197162.7
(22) Date of filing: 16.09.2021
(51) Int. Cl.: A61K 35/28, A61K 35/50, A61K 38/48, A61Q 19/00, C12N 5/0797, A61P 17/02, A61Q 19/08

(54) **FUNCTIONAL COMPOSITION COMPRISING EXOSOME-RICH CONDITIONED MEDIUM OF IMMORTALIZED STEM CELLS AND BOTULINUM TOXIN**

(30) Priority: 18.06.2021 KR 20210079371
(71) Applicant: Chungbuk National University Industry-Academic Cooperation Foundation, Cheongju-si, Chungcheongbuk-do 28644 (KR); Designed Cells Co., Ltd., Cheongju-si, Chungcheongbuk-do 28576 (KR)
(72) Inventor: KIM, Yun Bae, 30092 Sejong-si (KR); CHOI, Ehn Kyoung, 34053 Daejeon (KR); KIM, Tae Myoung, 28498 Cheongju-si (KR)
(74) Representative: Gottschald Patentanwälte Partnerschaft mbB

(57) **Abstract**

Disclosed herein is a functional composition comprising an exosome-rich conditioned medium of immortalized stem cells and botulinum toxin. Functioning to prevent the degradation of collagen and elastin, promote the synthesis of collagen and elastin, reduce wrinkles, and stimulate cell regeneration, the functional composition according to the present disclosure can find advantageous applications as a functional material for wrinkle reduction, skin regeneration, improvement in skin elasticity, and prevention of skin aging and wrinkle generation or in use for treating a wound.

## Description

### BACKGROUND OF THE INVENTION

### 1. Field of the invention

The present disclosure relates to a functional composition comprising an exosome-rich conditioned medium of immortalized stem cells and botulinum toxin.

### 2. Description of the Prior Art

The skin is a primary organ that protects and defends the human body. The skin consisting of the epidermis, dermis, and subcutaneous tissue is the largest organ covering the entire body surface. Of the dermal constituents, the stratum corneum, which is the outermost layer of the epidermis, is composed of keratins and lipids, functioning to protect the human body from infiltration of harmful substances, dehydration, bacterial infection, and external stress. The stratum corneum is renewed at regular intervals of two weeks.

The skin is frequently damaged by various stress factors or stimuli such as ultraviolet light. Such skin damage-inducing factors cause destruction of the keratin and lipid layers, which leads to a rapid increase in percutaneous water loss. In addition, dry skin, wrinkling, pruritus, and inflammation subsequent to bacterial infection may also be incurred. In response to external stimuli, human keratinocytes can release various cytokines IL-6, IL-8, TNF-α, and so on, starting with IL-1α. These cytokines mediate skin irritation or topical inflammatory reactions.

Particularly, repetition of damages by external stimuli, such as ultraviolet light, on the stratum corneum or delay of restoration to normal stratum cornea accelerates skin aging which may develop into skin diseases. After external damage, rapid restoration of the stratum cornea can reduce skin dry, skin aging, pruritus, and the like, and can be achieved by promoting skin regeneration, which induces normal differentiation and proliferation of human keratinocytes. Skin regeneration is determined by healing duration which varies depending on the size or degree of wounds on the skin. Wound healing is depicted in a discrete timeline of four developmental processes divided into hemostasis, inflammation, cell proliferation, and tissue remodeling, which occur in a controlled manner requiring various cellular interactions, growth factors, and cytokines.

In this regard, Korean Patent Number 10-2214378 relates to a composition for skin regeneration, wrinkle reduction, anti-inflammation, or skin whitening, and discloses that complanatoside A functions to increase a total amount of collagen in fibroblasts in the skin, suppress collagenase activity, improve a skin regeneration or anti-wrinkle effect, inhibit nitrogen oxide production to exhibit an anti-inflammatory effect, and decrease a total amount of melanin to exert a skin whitening effect and, as such, can be used in a pharmaceutical composition, a medicinal agent for external use, a cosmetic composition, or a health functional food.

### SUMMARY OF THE INVENTION

An aspect of the present disclosure is to provide a functional composition comprising an exosome-rich conditioned medium of immortalized stem cells and botulinum toxin.

Another aspect of the present disclosure is to provide a use of an exosome-rich conditioned medium of immortalized stem cells and botulinum toxin.

In accordance with an aspect thereof, the present disclosure provides a functional composition comprising an exosome-rich conditioned medium (ERCM) of immortalized stem cells and botulinum toxin.

In accordance with another aspect thereof, the present disclosure provides a pharmaceutical composition comprising an exosome-rich conditioned medium (ERCM) of immortalized stem cells and botulinum toxin as active ingredients for preventing or treating a wound.

In accordance with a further aspect thereof, the present disclosure provides a kit for preventing or treating a wound, the kit comprising: a first composition comprising an exosome-rich conditioned medium of immortalized stem cells as an active ingredient; and a second composition comprising botulinum toxin as an active ingredient.

In accordance with still another aspect thereof, the present disclosure provides improving method of skin condition comprising a step of administering an exosome-rich conditioned medium of immortalized stem cells and botulinum toxin to a subject.

In accordance with a still further aspect thereof, the present disclosure provides a use of a combination of an exosome-rich conditioned medium of immortalized stem cells and botulinum toxin in preparing a composition for improving skin condition.

In accordance with yet another aspect thereof, the present disclosure provides a method for preventing, alleviating, or treating a wound, the method comprising a step of administering an exosome-rich conditioned medium of immortalized stem cells and botulinum toxin to a subject.

In accordance with a yet further aspect thereof, the present disclosure provides a use of a combination of an exosome-rich conditioned medium of immortalized stem cells and botulinum toxin in preparing a medicine for preventing, alleviation, or treating a wound.

Functioning to prevent the degradation of collagen and elastin, promote the synthesis of collagen and elastin, reduce wrinkles, and stimulate cell regeneration, the functional composition according to the present disclosure can find advantageous applications as a functional material for wrinkle reduction, skin regeneration, improvement in skin elasticity, and prevention of skin aging and wrinkle generation or in use for treating a wound.

### BRIEF DESCRIPTION OF THE DRAWINGS

The above and other aspects, features and advantages of the present disclosure will be more apparent from the following detailed description taken in conjunction with the accompanying drawings, in which:
FIG. 1 is an image identifying CD9 which is an exosome marker present in an exosome-rich conditioned medium prepared in an embodiment of the present disclosure;
FIG. 2 is a graph showing contents of growth factors and neurotrophic factors present in an exosome-rich conditioned medium prepared in an embodiment of the present disclosure (* indicates a significant difference relative to the normal medium (CM));
FIG. 3 is a graph showing inhibition effects of an exosome-rich conditioned medium and botulinum toxin type A prepared in an embodiment of the present disclosure on the activity of the collagenase MMP-1 (* indicates a significant difference relative to the normal activity);
FIG. 4 is a graph showing inhibition effects of an exosome-rich conditioned medium and botulinum toxin type A prepared in an embodiment of the present disclosure on the activity of the elastase MMP-12 (* indicates a significant difference relative to the normal activity);
FIG. 5 is a graph showing promotive effects of an exosome-rich conditioned medium and botulinum toxin type A prepared in an embodiment of the present disclosure on the synthesis of collagen (* indicates a significant difference relative to the normal culture condition);
FIG. 6 is a graph showing promotive effects of an exosome-rich conditioned medium and botulinum toxin type A prepared in an embodiment of the present disclosure on the synthesis of elastin (* indicates a significant difference relative to the normal culture condition);
FIG. 7 is images showing reductive effects of an exosome-rich conditioned medium and botulinum toxin type A prepared in an embodiment of the present disclosure on skin wrinkles;
FIG. 8 is a plot quantitatively showing reductive effects of an exosome-rich conditioned medium and botulinum toxin type A prepared in an embodiment of the present disclosure on skin wrinkles (* indicates a significant difference relative to UVB-induced skin wrinkles); and
FIG. 9 is images showing regenerative effects of an exosome-rich conditioned medium and botulinum toxin type A prepared in an embodiment of the present disclosure on the skin.

### DETAILED DESCRIPTION OF THE EXEMPLARY EMBODIMENTS

Below, a detailed description will be given of the present disclosure.

The present disclosure provides a functional composition comprising an exosome-rich conditioned medium (ERCM) of immortalized stem cells and a botulinum toxin.

As used herein, the term "stem cells" refers to cells that are relatively undifferentiated cells capable of differentiating into cells of specific tissues. Potency that specifies the differentiation potential divides stem cells into pluripotent stem cells, multipotent stem cells, and unipotent stem cells. According to types of origin cells thereof, stem cells are also divided into embryonic stem cells, adult stem cells, and induced pluripotent stem cells (iPSC) created from human somatic cells. Specifically, the stem cells according to the present disclosure may be adult stem cells. The term "stem cells", as used herein, refers to undifferentiated cells that are found in adult tissues or organs and can self-renew and differentiate to desired cells. By way of example, the stem cells according to the present disclosure may be amniotic fluid adult stem cells.

As used herein, the term "immortalized cells" refers to cells that can proliferate indefinitely in cell passage processes without undergoing normal cellular senescence and death. Generally, with the progression of passages, the proliferative potential of cells decreases and contents of effective ingredients in the medium decrease, but immortalized cells can make constant maintenance of proliferate potential and contents of effective ingredients in the medium. The immortalized stem cells according to the present disclosure can be prepared using a method known in the art. In addition, the preparation may be appropriately modified by a person skilled in the art to maximize desired features of immortalized stem cells. For example, the immortalized stem cells according to the present disclosure may be human amniotic fluid stem cells. Specifically, the immortalized stem cells are a cell line, designated CBNU-AFSC for biomaterial classification, which was deposited under accession number KCTC12634BP on July 25, 2014 to the Korean Collection for Cell Types located at 181 Ipsin-gil. Jeongeup-si, Jeollabuk-do 305-806, Korea.

As used herein, the term "exosome-rich conditioned medium" (ERCM) refers to a medium in which immortalized stem cells with such above-described characteristics have been cultured. Methods for obtaining culture media increased exosome contents by culturing immortalized stem cells are well known in the art. The term "exosomes", as used herein, refers to membrane-bound extracellular vesicles (EVs) secreted from various kinds of cells. Exosomes generally range in average size from about 50 to 200 nm and are bound to cells or tissues other than their origin to perform various functions such as transfer of their cargos including membrane components, proteins, RNA, etc. Exosomes can be identified using marker proteins included therein. The marker proteins may include any of marker proteins known in the art and particularly may be CD9.

For example, the exosome-rich conditioned medium according to the present disclosure may be a medium in which immortalized stem cells have been cultured in the presence of TNF-α. In this regard, TNF-α may be contained at a concentration of 3 to 100 ng/ml, 3 to 90 ng/ml, 3 to 80 ng/ml, 3 to 60 ng/ml, 10 to 100 ng/ml, 10 to 90 ng/ml, 10 to 80 ng/ml, 10 to 70 ng/ml, 10 to 60 ng/ml, 20 to 100 ng/ml, 20 to 90 ng/ml, 20 to 80 ng/ml, 20 to 70 ng/ml, 20 to 60 ng/ml, 30 to 100 ng/ml, 30 to 90 ng/ml, 30 to 80 ng/ml, 30 to 70 ng/ml, 30 to 60 ng/ml, 40 to 100 ng/ml, 40 to 90 ng/ml, 40 to 80 ng/ml, 40 to 70 ng/ml, or 40 to 60 ng/ml.

In addition, the exosome-rich conditioned medium may be obtained by culturing immortalized stem cells in a hypoxic condition. The hypoxic condition is accounted for by an oxygen concentration lower than about 20%, the average oxygen concentration of the ordinary atmosphere. For instance, the hypoxic condition may mean an oxygen concentration of 10% or less, 0.1 to 10%, 0.1 to 8%, 0.1 to 5%, 0.1 to 3%, 0.5 to 10%, 0.5 to 8%, 0.5 to 5%, 0.5 to 3%, 1 to 10%, 1 to 8%, 1 to 5%, or 1 to 3%. Furthermore, the culturing may be performed for 1 to 80 hours, 1 to 70 hours, 1 to 60 hours, 1 to 50 hours, 10 to 80 hours, 10 to 70 hours, 10 to 60 hours, 10 to 50 hours, 20 to 80 hours, 20 to 70 hours, 20 to 60 hours, 20 to 50 hours, 30 to 80 hours, 30 to 70 hours, 30 to 60 hours, 30 to 50 hours, 40 to 80 hours, 40 to 70 hours, 40 to 60 hours, or 40 to 50 hours.

The exosome-rich conditioned medium according to the present disclosure may be filtered or concentrated, as necessary, to remove impurities therefrom.

The exosome-rich conditioned medium may contain a growth factor and a neurotrophic factor. For example, the growth factor and neurotrophic factor may be any of the factors known in the art. Particularly, the growth factor and neurotrophic factor may be at least one selected from the group consisting of bFGF (basic fibroblast growth factor), EFG (elongation factor G), IGF (insulin-like growth factor), VEGF (vascular endothelial growth factor), PDGF (platelet-derived growth factor), and TGF-β (transforming growth factor-β). More particularly, the growth factor and neurotrophic factor may include bFGF, EFG, IGF, VEGF, PDGF, and TGF-β.

As used herein, the term "botulinum toxin" refers to a neurotoxin protein with a molecular weight of about 150 kDa, produced by the Gram-positive anaerobic bacterium *Clostridium botulinum.* There are seven main serotypes of botulinum toxin, named types A, B, C, D, E, F, and G, which may be different in acting sites on the nerve. Particularly, the botulinum toxin may be at least one selected from the group consisting of botulinum toxin types A, B, C, D, E, F, and G and more particularly may be botulinum toxin type A.

Botulinum toxin type A can block the release of the neurotransmitter acetylcholine from nerve endings from the cholinergic nervous system dispersed in the skin to the neuromuscular junction.

Irrespective of whether it is isolated, extracted, or commercially available, any botulinum toxin may be employed. Also, a variant or a fusion protein of botulinum toxin falls within the scope of the botulinum toxin useful in the present disclosure. The variant of botulinum toxin may include a truncated form as long as it retains the activity of botulinum toxin. In addition, the variant may result from a chemical or functional modification on one or more amino acid residues of botulinum toxin. By way of example, the modification may be substitution, deletion, or insertion of one or more amino acid residues.

The composition according to the present disclosure may be contained botulinum toxin in an amount of 1 to 200 units, 1 to 150 units, 1 to 100 units, 1 to 50 units, 1 to 30 units, 1 to 10 units, 3 to 200 units, 3 to 150 units, 3 to 100 units, 3 to 50 units, 3 to 30 units, or 3 to 10 units.

Employing botulinum toxin in mixture or combination with an exosome-rich conditioned medium of immortalized stem cells, the composition according to the present disclosure can exhibit a significant effect even with a small amount of botulinum toxin and can prevent side effects caused by an excess of botulinum toxin, such as skin sagging, focal paralysis, muscle function loss, etc. Particularly, the wording "botulinum toxin in mixture with an exosome-rich conditioned medium of immortalized stem cells" accounts for one composition containing an exosome-rich conditioned medium of immortalized stem cells and botulinum toxin together, and the wording "botulinum toxin in combination with an exosome-rich conditioned medium of immortalized stem cells" accounts for compositions that contain an exosome-rich conditioned medium of immortalized stem cells and botulinum toxin, respectively and may be administered in combination, simultaneously, sequentially, or in a reverse order.

The composition according to the present disclosure may be a composition for improving skin condition. Particularly, the improving skin condition may be accounted for by ameliorating the skin that has been damaged by various stimuli such as ultraviolet light, aging, stress, etc. For example, the improving skin condition may be achieved by at least one function selected from the group consisting of skin regeneration, wrinkle reduction, improvement of skin elasticity, suppression of skin aging, and wrinkle prevention. In addition, the composition may be a health functional food, a cosmetic composition, or a topical agent.

That is, the composition according to the present disclosure may be a health functional food, a cosmetic composition, or a topical agent for skin care.

For the health functional food, the composition of the present disclosure as an active ingredient may be added alone to a base food, alone or together with a different food or a food ingredient. In this regard, the content of the active ingredient added may be determined according to the purpose of the health function food and may amount to 0.01 to 90 parts by weight, based on the total weight of the food.

No particular limitations are imparted to formulations and kinds of the health functional food. Particularly, the health functional food may be in the form of a tablet, a capsule, powder, granules, a liquid, and a pill. The health functional food may comprise various additional ingredients including a flavorant, a sweetener, and a natural carbohydrate. The sweetener may be natural or synthetic. Examples of the natural sweetener include thaumatin, a stevia extract, etc. The synthetic sweetener may be exemplified by saccharin, and aspartame. The natural carbohydrates may be monosaccharides, disaccharides, polysaccharides, oligosaccharides, and sugar alcohols.

The health functional food of the present disclosure may further comprise a nutrient, a vitamin, an electrolyte, a flavorant, a colorant, pectic acid and a salt thereof, an alginic acid and a salt thereof, an organic acid, a protective colloidal thickener, a pH controller, a stabilizer, a preservative, glycerin, an alcohol, etc. plus the additional ingredient described above. These ingredients may be used separately or in combination. The additives may be used in an amount of 0.01 to 0.1 parts by weight, based on 100 parts by weight of the composition of the present disclosure.

Furthermore, the cosmetic composition may comprise the composition of the present disclosure as an active ingredient in an amount of 0.1 to 50 % by weight and particularly in an amount of 1 to 10 % by weight. The cosmetic composition may be applied directly to the skin to improve skin care.

The cosmetic composition of the present disclosure can be formulated into any form that is typically accepted in the art. Examples of the cosmetic formulation include a solution, a suspension, an emulsion, a paste, a gel, a cream, a lotion, a powder, a soap, a surfactant-containing cleanser, an oil, a powdered foundation, an emulsified foundation, a wax foundation, and spray. Particularly, the cosmetic composition of the present disclosure may be in the form of soft lotion, nutrition lotion, nutrition cream, massage cream, essence, eye cream, cleansing cream, cleansing foam, cleansing water, a pack, spray, or powder.

For a paste, a cream, or a gel, the cosmetic composition of the present disclosure may include animal oil, vegetable oil, wax, paraffin, starch, tragacanth, a cellulose derivative, polyethylene glycol, silicone, bentonite, silica, talc, zinc oxide, or a mixture thereof as a carrier. For a powder or spray form, the cosmetic composition may include lactose, talc, silica, aluminum hydroxide, calcium silicate, polyamide powder, or a mixture thereof as a carrier. In particular, when formulated into a spray, the cosmetic composition may further include chlorofluorohydrocarbon, propane/butane, dimethyl ether, etc.

For a solution or an emulsion, the cosmetic composition of the present disclosure may include a solvent, a solubilizer, an emulsifier, or a mixture thereof as a carrier. Examples of the carrier include water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, and sorbitan fatty acid ester.

When formulated into a suspension, the cosmetic composition of the present disclosure may include, as a carrier, a liquid-phase diluent such as water, ethanol or propylene glycol; a suspending agent such as ethoxylated isostearyl alcohol, polyoxyethylene sorbitol ester and polyoxyethylene sorbitan ester; microcrystalline cellulose; aluminum metahydroxide; bentonite; agar; and tragacanth; or a mixture thereof. For a surfactant-containing cleansing formulation, the cosmetic composition of the present disclosure may include aliphatic alcohol sulfate, aliphatic alcohol ether sulfate, sulfosuccinic monoester, isethionate, an imidazolinum derivative, methyltaurate, sarcosinate, fatty acid amide ether sulfate, alkyl amidobetain, aliphatic alcohol, fatty acid glyceride, fatty acid diethanolamide, vegetable oil, a lanolin derivative, ethoxylated glycerol fatty acid ester, or a mixture thereof as a carrier.

In addition to the carrier component, the cosmetic composition of the present disclosure may include an antioxidant, a stabilizer, a solubilizer, a moisturizer, a pigment, a fragrance, a sun block, a color-developing agent, a surfactant, or a mixture thereof as an auxiliary agent. So long as it is usually used for preparing a cosmetic composition, any auxiliary agent may be available in the present disclosure.

Furthermore, the topical agent may include a pharmaceutically acceptable carrier and excipient. Within the scope of the carrier and excipient may be a preservative, a stabilizer, a hydrating agent, an emulsifier, and a buffer. Specifically, the excipient may be lactose, dextrin, starch, mannitol, sorbitol, glucose, saccharose, microcrystalline cellulose, gelatin, polyvinylpyrrolidone, or a mixture thereof. The topical agent may be appropriately prepared according to a method well known in the art. The topical agent may be prepared into a form of a powder, a gel, an ointment, a cream, a liquid, or an aerosol.

Another aspect of the present disclosure provides a pharmaceutical composition comprising an exosome-rich conditioned medium of immortalized stem cells and botulinum toxin as active ingredients for preventing or treating of a wound.

As active ingredients in the pharmaceutical composition according to the present disclosure, the exosome-rich conditioned medium of immortalized stem cells and the botulinum toxin are characterized as described above. The stem cells may be human amniotic fluid stem cells, and particularly, the immortalized stem cells deposited under accession number KCTC12634BP. In addition, the exosome-rich conditioned medium may be a medium in which the immortalized stem cells have been cultured in the presence of TNF-α. In addition, the exosome-rich conditioned medium may be a medium cultured in a hypoxic condition.

The botulinum toxin may be at least one selected from the group consisting of serotypes A, B, C, D, E, F, and G and particularly may be botulinum toxin type A.

As used herein, the term "wound" refers to a damaged condition of the body and is intended to encompass pathological conditions in which the tissues constituting the inside or outside of the body, for example, skin, a muscle, a nerve tissue, a bone, a soft tissue, an internal organ, or a vascular tissue is torn or destroyed. In detail, examples of the wound may include an abrasion, a laceration, a stab, an incision, an avulsion, a pressure sore, a bedsore, radiation-induced tissue destruction, a penetration wound, a gunshot wound, a burn, frostbite, a surgical wound, a post-plastic surgery sutured site, and a chemical wound.

The exosome-rich conditioned medium of immortalized stem cells and the botulinum toxin may be formulated separately or in combination.

Furthermore, the exosome-rich conditioned medium of immortalized stem cells and botulinum toxin used as active ingredients in the pharmaceutical composition according to the present disclosure may be contained in an amount of 0.1 to 95 % by weight, based on the total weight of the composition. In addition to the active ingredients, the pharmaceutical composition of the present disclosure may further comprise at least one ingredient functionally identical or similar thereto.

The pharmaceutical composition of the present disclosure may include a carrier, a diluent, an excipient, or a mixture thereof typically used in biological preparations. So long as it is suitable to transfer the composition into the body, any pharmaceutically acceptable carrier can be used. In detail, the carrier may be saline, sterilized water, Ringer's solution, dextrose solution, maltodextrin solution, glycerol, ethanol, or a mixture thereof. For more details, reference may be made to Merck Index, 13th ed., Merck & Co. Inc. If necessary, a general additive such as an antioxidant, a buffer, and a bacteriostatic agent may be additionally employed.

When formulated, the composition may be added with typically used diluents or excipients such as fillers, extenders, binders, humectants, disintegrants, surfactants, and so on.

The composition of the present disclosure may be formulated into oral dosage forms or parenteral dosage forms. Oral dosage forms may be solid formulations or liquid formulations. The solid formulations for oral administration may be tablets, pills, powder, granules, capsules, or troches. These solid formulations are prepared by mixing the composition with one or more suitable excipients such as starch, calcium carbonate, sucrose, lactose, gelatin, or a mixture thereof. Further, the solid formulations may contain lubricants such as magnesium stearate and talc. On the other hand, the liquid formulations may be suspensions, solutions, emulsions, or syrups. The liquid formulations may also contain excipients such as humectants, sweeteners, aromatics, and preservatives.

Formulations for parenteral administration can include injections, suppositories, powders for respiratory inhalation, aerosols for spray, powders, and creams. The injection may include sterilized aqueous solutions, non-aqueous solvents, suspension solvents, emulsions, and the like. In this regard, propylene glycol, polyethylene glycol, vegetable oil such as olive oil, injectable ester such as ethylolate, may be used as the non-aqueous solvent or suspension solvent.

Another aspect of the present disclosure provides a kit for prevention or treatment of a wound, the kit comprising: a first composition comprising an exosome-rich conditioned medium of immortalized stem cells as an active ingredient; and a second composition comprising botulinum toxin as an active ingredient.

As active ingredients in the therapeutic kit according to the present disclosure, the exosome-rich conditioned medium of immortalized stem cells and the botulinum toxin are characterized as described above. The stem cells may be human amniotic fluid stem cells, and particularly, the immortalized stem cells deposited under accession number KCTC12634BP. In addition, the exosome-rich conditioned medium may be a medium in which the immortalized stem cells have been cultured in the presence of TNF-α. In addition, the exosome-rich conditioned medium may be a medium obtained in a hypoxic condition.

The botulinum toxin may be at least one selected from the group consisting of serotypes A, B, C, D, E, F, and G and particularly may be botulinum toxin type A.

In addition, the wound may be characterized as described above.

The first composition and the second composition may exhibit the same characteristics as in the pharmaceutical composition described above, and may be administered in combination, simultaneously, sequentially, or in a reverse order. In detail, the exosome-rich conditioned medium of immortalized stem cells and the botulinum toxin may be simultaneously administered. Alternatively, the exosome-rich conditioned medium of immortalized stem cells may be administered prior to the botulinum toxin. In another option, the botulinum toxin may be administered, followed by the exosome-rich conditioned medium of immortalized stem cells. In the context of administering the exosome-rich conditioned medium of immortalized stem cells and the botulinum toxin in the sequential order or the reverse order, administration intervals can be appropriately adjusted by a person skilled in the art.

Another aspect of the present disclosure provides improving method of skin condition comprising a step of administering an exosome-rich conditioned medium of immortalized stem cells and botulinum toxin to a subject.

As active ingredients in the improving method of skin condition according to the present disclosure, the exosome-rich conditioned medium of immortalized stem cells and the botulinum toxin are characterized as described above. The stem cells may be human amniotic fluid stem cells, and particularly, the immortalized stem cells deposited under accession number KCTC12634BP. In addition, the exosome-rich conditioned medium may be a medium in which the immortalized stem cells have been cultured in the presence of TNF-α. In addition, the exosome-rich conditioned medium may be a medium cultured in a hypoxic condition.

The botulinum toxin may be at least one selected from the group consisting of serotypes A, B, C, D, E, F, and G and particularly may be botulinum toxin type A.

The subject may be a mammal and particularly a human.

Depending on purposes, the administration may be conducted through an oral or parenteral route. Examples of the parenteral administration include intraperitoneal, intrarectal, subcutaneous, intravenous, intramuscular, and intrapulmonary injections. Also, the parenteral administration may be application to the skin.

Another aspect of the present disclosure provides a use of an exosome-rich conditioned medium of immortalized stem cells and botulinum toxin in preparing a composition for improving skin condition.

The exosome-rich conditioned medium of immortalized stem cells and the botulinum toxin which are used in preparing a composition for improving skin condition according to the present disclosure are characterized as described above. The stem cells may be human amniotic fluid stem cells, and particularly, the immortalized stem cells deposited under accession number KCTC12634BP. In addition, the exosome-rich conditioned medium may be a medium in which the immortalized stem cells have been cultured in the presence of TNF-α. In addition, the exosome-rich conditioned medium may be a medium cultured in a hypoxic condition.

The botulinum toxin may be at least one selected from the group consisting of serotypes A, B, C, D, E, F, and G and particularly may be botulinum toxin type A.

Another aspect of the present disclosure provides a method for preventing, alleviation, or treating a wound, the method comprising a step of administering an exosome-rich conditioned medium of immortalized stem cells and botulinum toxin to a subject.

The exosome-rich conditioned medium of immortalized stem cells and the botulinum toxin which are used in the method for preventing, alleviation, or treating a wound according to the present disclosure are characterized as described above. The stem cells may be human amniotic fluid stem cells, and particularly, the immortalized stem cells deposited under accession number KCTC12634BP. In addition, the exosome-rich conditioned medium may be a medium in which the immortalized stem cells have been cultured in the presence of TNF-α. In addition, the exosome-rich conditioned medium may be a medium cultured in a hypoxic condition.

The botulinum toxin may be at least one selected from the group consisting of serotypes A, B, C, D, E, F, and G and particularly may be botulinum toxin type A.

The subject may be a mammal and particularly a human.

Depending on purposes, the administration may be conducted through an oral or parenteral route. Examples of the parenteral administration include intraperitoneal, intrarectal, subcutaneous, intravenous, intramuscular, and intrapulmonary injections. Also, the parenteral administration may be application to the skin. A pharmaceutically effective amount may be administered and may vary depending on various factors including the types and severity of disease, drug activity, the patient's sensitivity to the drug, the time of administration, the route of administration, the duration of treatment, and the drug used in combination. However, the active ingredient of the present disclosure may be administered at a dose of 0.0001 to 1,000 mg/kg and particularly at a dose of 0.001 to 500 mg/kg and one or more times a day for a desired effect.

The composition may be administered alone or in combination with other therapeutics. For combination administration, the administration may be conducted sequentially or simultaneously.

Another aspect of the present disclosure provides a use of an exosome-rich conditioned medium of immortalized stem cells and botulinum toxin in preparing a medicine for preventing, alleviating, or treating a wound according to the present disclosure.

The exosome-rich conditioned medium of immortalized stem cells and the botulinum toxin which are used in a medicine for preventing, alleviating, or treating a wound according to the present disclosure are characterized as described above. The stem cells may be human amniotic fluid stem cells, and particularly, the immortalized stem cells deposited under accession number KCTC12634BP. In addition, the exosome-rich conditioned medium may be a medium in which the immortalized stem cells have been cultured in the presence of TNF-α. In addition, the exosome-rich conditioned medium may be a medium cultured in a hypoxic condition.

The botulinum toxin may be at least one selected from the group consisting of serotypes A, B, C, D, E, F, and G and particularly may be botulinum toxin type A.

A better understanding of the present disclosure may be obtained through the following examples which are set forth illustrate but are not to be construed as the limit of, the present disclosure.

### EXAMPLE 1: Preparation of Exosome-Rich Conditioned Medium of Immortalized Stem Cells

An exosome-rich conditioned medium (ERCM) was obtained by culturing immortalized stem cells as follows.

First, amniotic fluid stem cells (accession number KCTC12634BP) were seeded at a density of 1x10⁶ cells/ml in DMEM (Dulbecco's modified Eagle's medium) and cultured at 37°C under a 5% CO₂ condition. After 24 hours, 50 ng/ml TNF-α was added to the medium. The oxygen concentration was lowered to 2% before culturing for an additional 48 hours. Then, the resulting culture medium was filtered to remove impurities such as cells, etc. Concentration by 30-fold at a low temperature afforded an exosome-rich conditioned medium (ERCM) of immortalized stem cells.

### COMPARATIVE EXAMPLE 1: Preparation of Culture Medium of Immortalized Stem Cells

Immortalized amniotic fluid stem cells (accession number KCTC12634BP) were cultured in DMEM at 37°C under a 5% CO₂ atmosphere. The culture medium was filtered to remove impurities such as cells, etc. Concentration by 30-fold at a low temperature afforded a normal medium (conditioned medium, CM) of immortalized stem cells.

### EXPERIMENTAL EXAMPLE 1: Assay for Exosome Content

The content of exosomes in the exosome-rich conditioned medium obtained above was analyzed by quantitating the expression of CD9, which is a marker of exosomes.

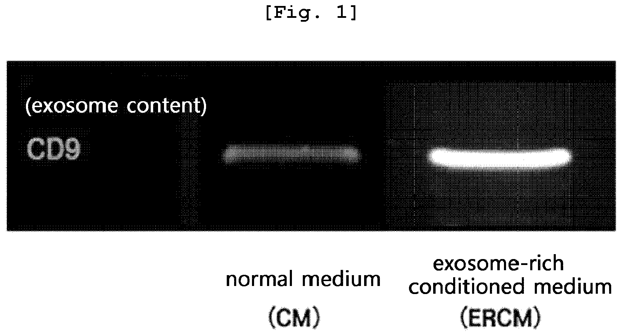

In brief, exosomes were isolated from 6 ml of the exosome-rich conditioned medium, using a total exosome isolation kit (Invitrogen), followed by an ordinary western blotting analysis for CD9. The result is shown in FIG. 1, with the normal medium (CM) of Comparative Example 1 serving as a control.

As shown in FIG. 1, the exosome-rich conditioned medium was about 50-fold higher in CD9 expression level than the normal medium (CM), with significance.

### EXPERIMENTAL EXAMPLE 2: Assay for Growth Factor and Neurotrophic Factor

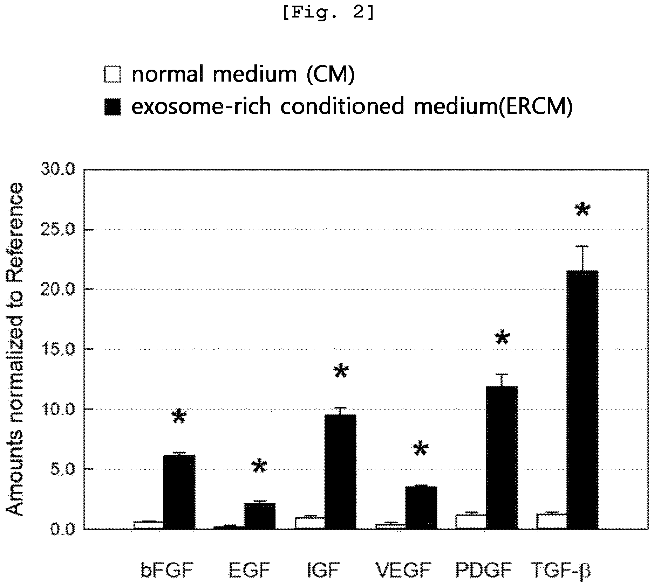

Growth factors and neurotropic factors in the exosome-rich conditioned medium obtained above were analyzed as follows. Experiments were conducted using an ordinary ELISA method. In brief, expression levels of bFGF (basic fibroblast growth factor), EFG (elongation factor G), IGF (insulin-like growth factor), VEGF (vascular endothelial growth factor), PDGF (platelet-derived growth factor), and TGF-β (transforming growth factor-β) proteins were measured. In this regard, the normal medium was employed as a control and measurements of the expression levels of growth factors and neurotrophic factors are depicted in FIG. 2.

As shown in FIG. 2, significantly high levels of the growth factors and neurotrophic factors were detected in the exosome-rich conditioned medium, compared to the normal medium. In detail, about 10-fold or higher increases were observed in the expression levels of bGFG, EFG, IGF, VEGF, and PDFG proteins, and especially, TGF-β increased by about 17-fold.

### EXPERIMENTAL EXAMPLE 3: Inhibitory Effect on Collagenase

Examination was made of the inhibitory effect of the exosome-rich conditioned medium on collagenase by inhibiting of MMP-1 (matrix metalloproteinase-1).

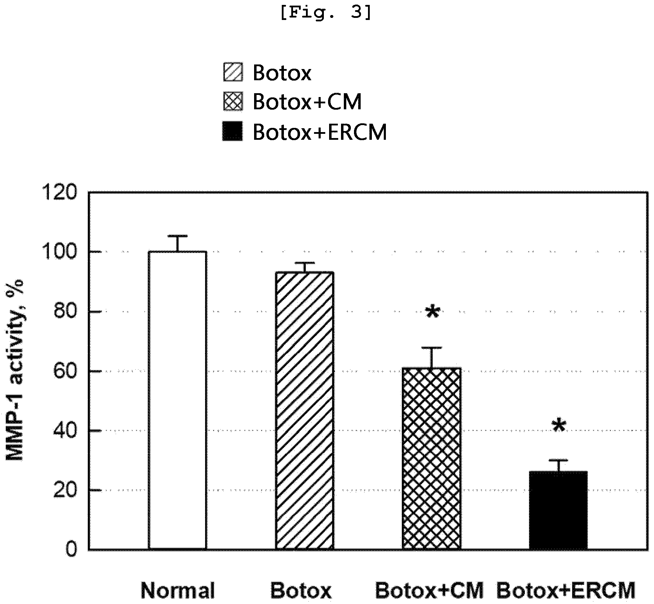

Briefly, a reaction mixture containing a substrate (MCA-Lys-Pro-Leu-Gly-Leu-DPA-Ala-Arg-NH₂) and recombinant human MMP-1 protein (Cat. #M9195, Sigma-Aldrich) was added with 100 µl/ml exosome-rich conditioned medium and 5 units of botulinum toxin type A (Medytox, Korea). Absorbance of the mixture was read for 5 min at 320 nm (excitation) and 405 nm (emission). For comparison, the reaction mixture was treated with no substances for a normal control, only botulinum toxin for a botulinum toxin-treated group, and botulinum toxin and normal medium (CM) for botulinum toxin + normal medium group. Activities of MMP-1 protein were calculated, and percentages based on the activity of the normal control are depicted in FIG. 3.

As shown in FIG. 3, botulinum toxin alone could not inhibit the activity of MMP-1 protein whereas botulinum toxin in combination with the exosome-rich conditioned medium significantly reduced the activity of MMP-1 protein, compared to botulinum toxin or the exosome-rich conditioned medium alone. In addition, this effect was significantly higher than that of a combination of botulinum toxin and the normal medium (CM).

### EXPERIMENTAL EXAMPLE 4: Inhibitory Effect on Elastase

Examination was made of the inhibitory effect of the exosome-rich conditioned medium on elastase by inhibiting of MMP-12 (matrix metalloproteinase-12).

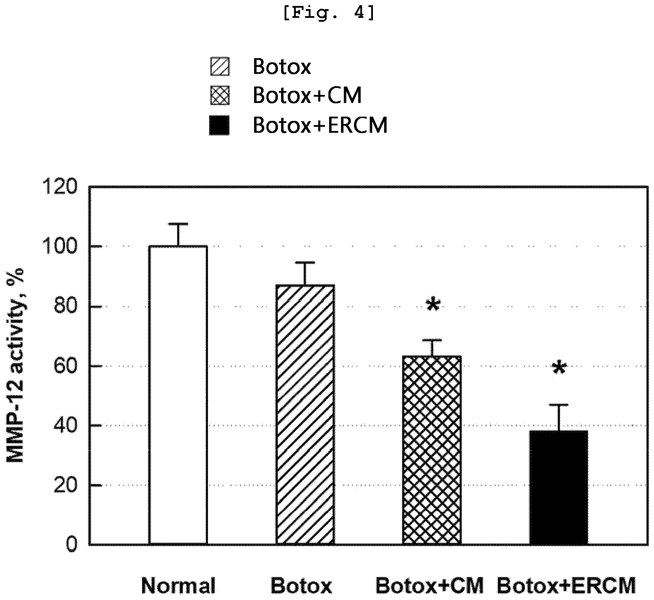

Briefly, a reaction mixture containing a substrate (N-succinyl-Ala-Ala-Ala-p-nitroanilide) and human neutrophil elastase (Cat. #MAK246, Sigma-Aldrich) was added with 100 µl/ml exosome-rich conditioned medium and 5 units of botulinum toxin type A (Medytox, Korea). Absorbance of the mixture was read for 5 min at 410 nm. For comparison, the reaction mixture was treated with no substances for a normal control; only botulinum toxin for a botulinum toxin-treated group; and botulinum toxin and normal medium (CM) for botulinum toxin + normal medium group. Activities of MMP-12 protein were calculated, and percentages based on the activity of the normal control are depicted in FIG. 4.

As shown in FIG. 4, botulinum toxin alone could not inhibit the activity of MMP-12 protein whereas botulinum toxin in combination with the exosome-rich conditioned medium significantly reduced the activity of MMP-12 protein, compared to botulinum toxin or the exosome-rich conditioned medium alone. In addition, this effect was significantly higher than that of a combination of botulinum toxin and the normal medium (CM).

### EXPERIMENTAL EXAMPLE 5: Promotive Effect on Collagen Synthesis

The exosome-rich conditioned medium obtained above was examined for promotive effect on collagen synthesis, as follows.

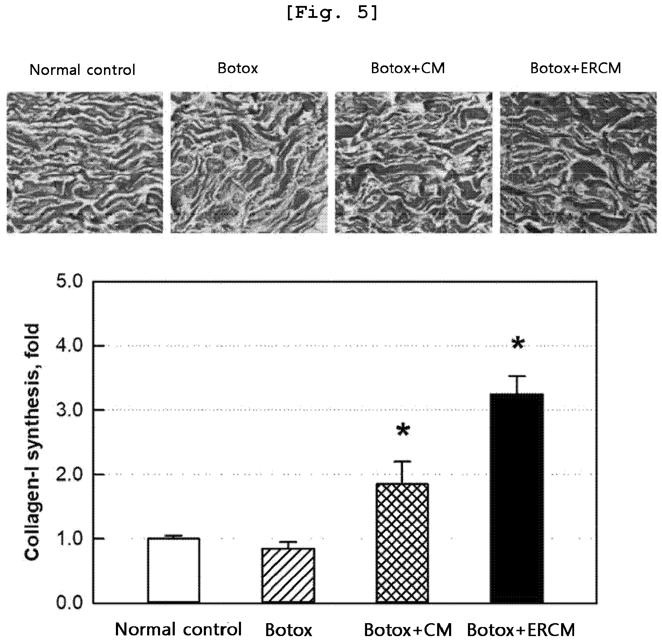

First, the human dermal fibroblast cell line CCD986SK (Thermo Fisher Scientific) was maintained by an ordinary method using medium 106. The cells thus prepared were seeded at a density of 5x10⁴ cells/well into 6-well plates, followed by adding 100 µl/ml exosome-rich conditioned medium and 5 units of botulinum toxin type A to each well. After incubation for 72 hours, collagen fibers of the cultured cells were stained with Masson's Trichrome and observed under a microscope. In addition, the cell culture supernatant was taken and subjected to ELISA using a goat anti-type 1 collagen-UNLB antibody. Absorbance was read at 450 nm to determine contents of type 1 collagen. For comparison, the cells were treated with no substances for a normal control; only botulinum toxin for a botulinum toxin-treated group; and botulinum toxin and normal medium (CM) for botulinum toxin + normal medium group. Microscopic images and ELISA analysis results are given in FIG. 5.

As shown in FIG. 5, botulinum toxin alone could not promote collagen synthesis whereas botulinum toxin in combination with the exosome-rich conditioned medium significantly increased collagen synthesis. In addition, this effect was significantly higher than that of a combination of botulinum toxin and the normal medium (CM).

### EXPERIMENTAL EXAMPLE 6: Promotive Effect on Elastin Synthesis

The exosome-rich conditioned medium obtained above was examined for promotive effect on elastin synthesis, as follows.

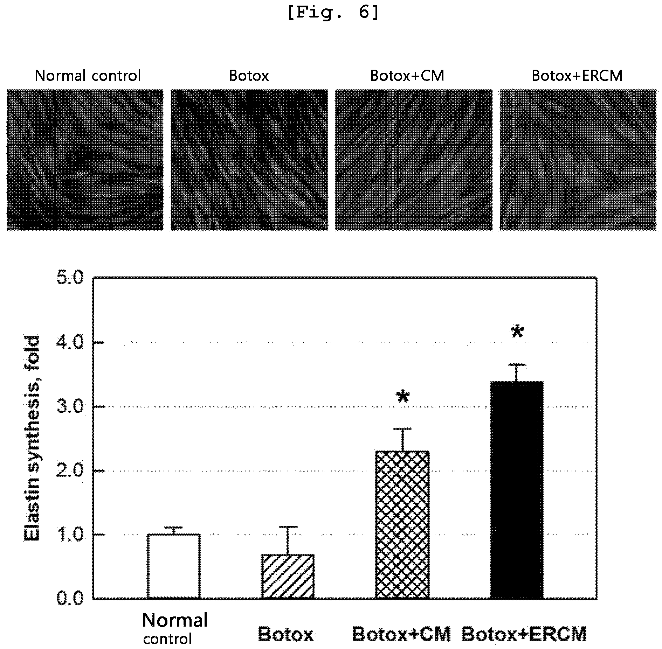

In brief, the cells were incubated for 7 days with the exosome-rich conditioned medium and botulinum toxin type A in the same condition and manner as in Experimental Example 3. Elastin fibers of the cultured cells were immunostained with a rabbit anti-human α-elastin antibody (Bio-Rad) in an ordinary manner and observed under a microscope. In addition, the cell culture supernatant was taken and subjected to ELISA using the same antibody. Absorbance was read at 450 nm to determine contents of elastin. For comparison, the cells were treated with no substances for a normal control, only botulinum toxin for a botulinum toxin-treated group, and botulinum toxin and normal medium (CM) for botulinum toxin + normal medium group. Microscopic images and ELISA analysis results are given in FIG. 6.

As shown in FIG. 6, botulinum toxin alone did not promote, but rather tended to suppress elastin synthesis whereas botulinum toxin in combination with the exosome-rich conditioned medium significantly increased elastin synthesis. In addition, this effect was significantly higher than that of a combination of botulinum toxin and the normal medium (CM).

### EXPERIMENTAL EXAMPLE 7: Reductive Effect on Skin Wrinkle

The exosome-rich conditioned medium obtained above was examined for reductive effect on skin wrinkles, as follows.

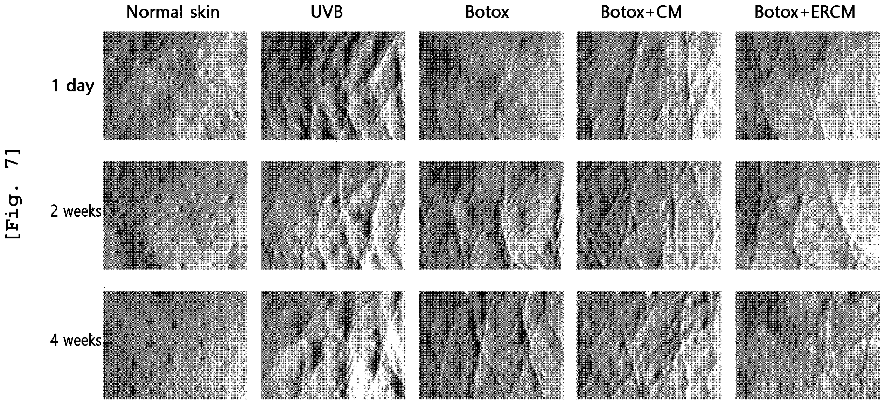

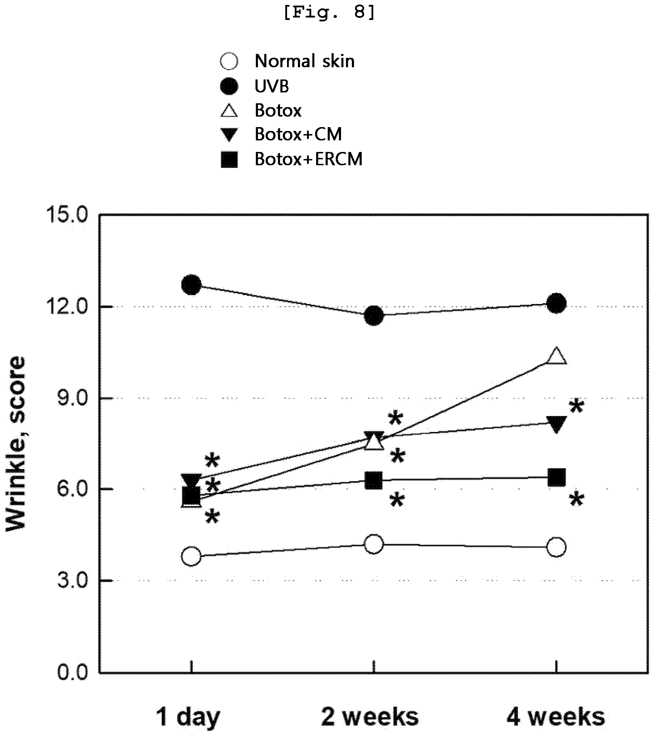

First, dorsal skins of male hairless mice (SKH-1) at 7 weeks of age were exposed to UVB from a UVB lamp (World Corporation). In brief, UVB was irradiated at one minimum erythema dose (MED) on the first week, at two MEDs on the second week, at three MEDs on the third week, and at four MEDs on the fourth to tenth week to cause skin wrinkles. The skin wrinkle-induced mice were each subcutaneously injected with 50 µl of the exosome-rich conditioned medium and 5 units of botulinum toxin type A. In this regard, injection was made at a total of 5 positions roughly depicting a circle, with a 1 cm distance between neighboring positions. The wrinkles were observed one day and two and four weeks after injection. For comparison, the mice were treated with no substances for a normal control; only UVB for a UVB-treated group; only botulinum toxin for a botulinum toxin-treated group; and botulinum toxin and normal medium (CM) for botulinum toxin + normal medium group. Subsequently, wrinkle replicas were constructed using a silicone polymer (CharmFlex^{®} Impression Material) and contrast images of wrinkles were taken using a camera, with light given at a fixed incident angle of 20 degrees. From the images, roughness was analyzed using an image analysis program (Skin Visiometer SV600 software). The microscopic images are given in FIG. 7 and wrinkle score analysis results are depicted in FIG. 8

As shown in FIGS. 7 and 8, the UVB-induced wrinkles were maintained until week 4 after UVB exposure, with the score of 11.7 points. When the botulinum toxin alone was applied, the wrinkles were greatly reduced, with the score measured at down to 5.6 points, one day after treatment, but wrinkles were regenerated, with the score increased to 7.5 and 10.3 points two and four weeks after treatment, respectively. In contrast, the regeneration of wrinkles was significantly blocked when the skin was treated with botulinum toxin in combination with the exosome-rich conditioned medium or the normal medium (CM). Particularly, when applied, a combination of the exosome-rich conditioned medium and botulinum toxin completely blocked the regeneration of wrinkles and exhibited a wrinkle reduction effect, with the score measured at 6.4 points even after 4 weeks.

Therefore, a combination of botulinum toxin and the exosome-rich conditioned medium, botulinum toxin exhibited significantly increased wrinkle reduction effects for a long period of time, compared to botulinum toxin alone or in combination with the normal medium (CM).

### EXPERIMENTAL EXAMPLE 8: Regenerative Effect of Skin

The exosome-rich conditioned medium obtained above was examined for regenerative effect on skin through wound scratch assay.

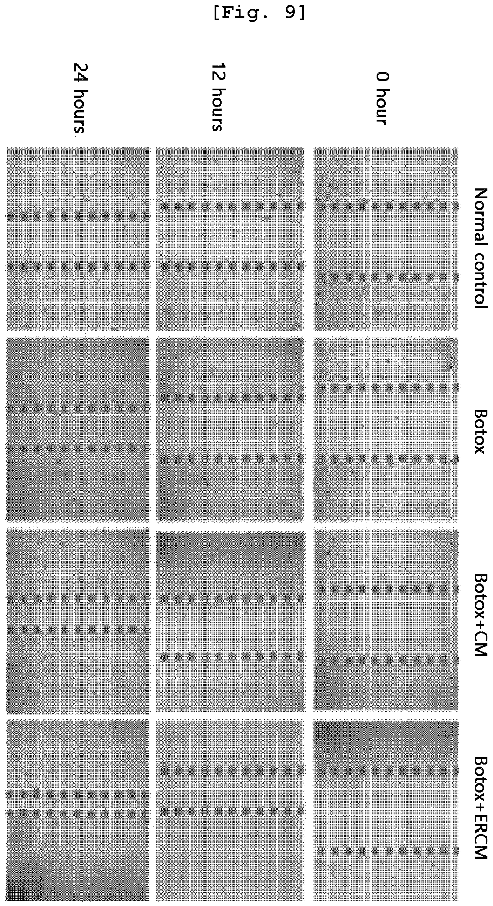

First, the human keratinocyte cell line NHEK (Guangdong BioCell Biotechnology Co., Ltd.) was maintained by an ordinary method using the EpiLife^{™} medium (Gibco). The cells thus prepared were seeded at a density of 3x10⁵ cells/well into 6-well plates, and a scratch was induced with a 100 µl pipette tip. The cells were cultured for 24 hours in the presence of 100 µl/ml exosome-rich conditioned medium and 5 units of botulinum toxin type A and observed. For comparison, the cells were treated with no substances for a normal control; only botulinum toxin for a botulinum toxin-treated group; and botulinum toxin and normal medium (CM) for botulinum toxin + normal medium group. The cells were quantitatively analyzed for degree of regeneration using a phase-contrast microscope. Images taken by the phase-contrast microscope are given in FIG. 9.

As shown in FIG. 9, cell regeneration was made by about 20% in the normal control and by about 35% in the botulinum toxin-treated group. In contrast, cell regeneration was significantly increased upon treatment with a combination of botulinum toxin and the exosome-rich conditioned medium or the normal medium (CM) and amounted to about 85 to 90% particularly in the co-existence of the exosome-rich conditioned medium and botulinum toxin.

Taken together, the data imply that simultaneous treatment with the exosome-rich conditioned medium and botulinum toxin remarkably increases cell regeneration.

## Claims

1. A functional composition, comprising an exosome-rich conditioned medium (ERCM) of immortalized stem cells and botulinum toxin.

2. The functional composition of claim 1, wherein the stem cells are human amniotic fluid-derived stem cells.

3. The functional composition of claim 1, wherein the immortalized stem cells are cells deposited under accession number KCTC12634BP.

4. The functional composition of claim 1, wherein the exosome-rich conditioned medium is obtained by culturing immortalized stem cells in presence of TNF-α.

5. The functional composition of claim 4, wherein the TNF-α is contained at a concentration of 3 to 100 ng/ml.

6. The functional composition of claim 1, wherein the exosome-rich conditioned medium is obtained by culturing immortalized stem cells at an oxygen concentration of 10% or less.

7. The functional composition of claim 6, wherein the exosome-rich conditioned medium is obtained by culturing immortalized stem cells at an oxygen concentration of 1 to 5%.

8. The functional composition of claim 1, wherein the botulinum toxin is at least one selected from the group consisting of botulinum toxin types A, B, C, D, E, F, and G.

9. The functional composition of claim 8, wherein the botulinum toxin is botulinum toxin type A.

10. The functional composition of claim 1, wherein the composition is for use in improving skin condition.

11. The functional composition of claim 10, wherein the composition performs skin care through at least one function selected from the group consisting of skin regeneration, wrinkle reduction, improvement of skin elasticity, prevention of skin aging, and prevention of wrinkle generation.

12. The functional composition of claim 1, wherein the composition is a health functional food, a cosmetic composition, or a topical agent.

13. Use of a combination of an exosome-rich conditioned medium of immortalized stem cells and botulinum toxin in preparing a medicine for preventing, alleviation, or treating a wound.

14. A kit for preventing or treating a wound, the kit comprising:
a first composition comprising an exosome-rich conditioned medium of immortalized stem cells as an active ingredient; and
a second composition comprising botulinum toxin as an active ingredient.

15. The kit of claim 14, wherein the exosome-rich conditioned medium of immortalized stem cells and the botulinum toxin are administered in combination, simultaneously, sequentially, or in a reverse order.
